# EUROPÄISCHE PATENTSCHRIFT

(11) **EP 0 924 217 B1**
(45) Veröffentlichungstag und Bekanntmachung des Hinweises auf die Patenterteilung: **17.03.2004**
(21) Anmeldenummer: 98123152.5
(22) Anmeldetag: 04.12.1998
(51) Int. Cl.: C07F 9/11, A61K 7/48

(54) **Phosphorsäurealkylester**
Phosphoric acid esters
Esters d'acides phosphoriques

(30) Priorität: 18.12.1997 DE 19756373
(43) Veröffentlichungstag der Anmeldung: 23.06.1999
(73) Patentinhaber: Clariant GmbH, 65929 Frankfurt am Main (DE)
(72) Erfinder: Turowski-Wanke, Angelika, Dr., 65779 Kelkheim (DE); Löffler, Matthias, Dr., 65527 Niedernhausen (DE)

(56) Entgegenhaltungen:
- EP-A- 0 227 012
- EP-A- 0 265 702
- WO-A-91/14693
- DE-A- 2 040 350
- DE-A- 2 209 819
- DE-A- 2 601 601
- DE-A- 2 708 746
- GB-A- 1 548 985
- GB-A- 2 133 316
- US-A- 2 822 290
- US-A- 3 397 150
- US-A- 3 816 346
- DE POLO K F ET AL: "Cetylphosphates as cosmetic emulsifiers" DRUG COSMET. IND. (DCINAQ,00126527);1989; VOL.145 (3); PP.26, 28, 30, 34, 82, 84, XP002097154 L. Givaudan et Cie;Cosmet. Spec. Dev. Lab.; Vernier; Switz. (CH)

## Beschreibung

Die Erfindung betrifft kosmetische und pharmazeutische Emulsionen enthaltend als Emulgator Phosphorsäurealkylester, vorzugsweise deren Mischungen. Diese Ester zeichnen sich aus durch eine sehr effektive Erniedrigung der Grenzflächenspannung, hohe Stabilität auch bei Temperaturbelastung und geringe Empfindlichkeit gegenüber Elektrolyten und Säuren.

Die Verwendung von Emulgatoren zur Herstellung von Cremes, Lotionen, Salben etc., die mehrere nicht miteinander mischbare Substanzen (z.B. Wasser, Öl, organische und anorganische Bestandteile) enthalten, ist lange bekannt. Als Emulgatoren wirken Tenside, z.B. Seifen von Alkalimetallen und Alkanolamine, Mono- und Diglycerinester von Fettsäuren, aber auch bestimmte Naturstoffe (z.B. Lecithine, Wachse) und anorganische Stoffe (z.B. Bentonite).

In EP-B-0 201 040 wird das Emulgiervermögen von Metallsalzen von Dialkylphosphaten und in EP-B-0 227 012 das von Mono-Phosphorsäureestern in Form von Alkalisalzen, Aminosäuresalzen oder Alkanolaminsalzen beschrieben.

Auch die in GB-A-2 139 112 und EP-A-265 702 als Emulgator beschriebenen Phosphorsäureester können als Alkali-, Aminosäure- oder Alkanolaminsalz vorliegen. Die Verwendung von C₁-C₃-Alkylammoniumsalzen dieser Phosphorsäureester als Emulgator ist in US-A-4 139 485 beschrieben.

Es wurde gefunden, daß sich Alkylphosphorsäureester in Form ihrer Fettalkylaminsalze universell zur Herstellung nicht nur von Öl- in-Wasser-Emulsionen, sondern auch für Wasser-in-Öl-Emulsionen eignen.

Gegenstand der Erfindung sind kosmetische und pharmazeutische Emulsionen enthaltend als Emulgator Phosphorsäurealkylester, insbesondere deren Mischungen, der Formel 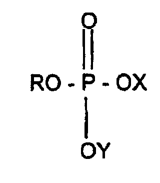 worin R C₁₂-C₂₂-, vorzugsweise C₁₄-C₁₈-Alkyl bedeutet, X eine Gruppe der Formeln NH₃R¹, NH₂R²R³ oder NHR⁴R⁵R⁶ bedeutet, Y die gleiche Bedeutung wie R oder die gleiche Bedeutung wie X hat und R¹ C₈-C₂₂-Alkyl, R² und R³ C₁-C₂₂-Alkyl oder Cyclohexyl, R⁴, R⁵ und R⁶ C₁-C₄-Alkyl bedeuten.

Die Herstellung dieser Phosphorsäurealkylester erfolgt nach an sich bekannten Verfahren durch Umsetzung von Tetraphosphordecaoxid und Fettalkoholen im ungefähren Molverhältnis 1:1 und nachfolgende Salzbildung mit einem Amin der Formeln NH₂R¹, NHR²R³ oder NR⁴R⁵R⁶ wobei R¹, R², R³, R⁴, R⁵ und R⁶ die oben genannte Bedeutung haben. Bei diesem Herstellverfahren fallen die Phosphorsäureester als ein Gemisch von im wesentlichen 30 bis 60 Gew.-% Mono- und 30 bis 60 Gew.-% Di-ester mit geringen Anteilen des korrespondierenden Tri-esters (0 bis 10 Gew.-%) an. Bevorzugt liegen die Phosphorsäureester als Gemische vor mit 30 bis 50 Gew.-% Mono-ester, 40 bis 60 Gew.-% Di-ester und 0 bis 2 Gew.-% Tri-ester. Normalerweise sind die Anteile von Mono- und Di-ester etwa gleich groß.

In einer bevorzugten Ausführungsform enthalten die erfindungsgemäßen Emulsionen Phosphorsäurealkylester, die 0 bis 10 Gew.-% des korrespondierenden Triesters enthalten.

Der Rest R kann geradkettig oder β-verzweigt sein. Bei den geradkettigen Fettalkoholen handelt es sich vorzugsweise um Gemische von Fettalkoholen, die sich von nativen Fettsäuren ableiten und dementsprechend Mischungen verschiedener Kettenlänge darstellen und daneben noch mehr oder weniger große Anteile ungesättigter Fettalkohole enthalten. Bei den β-verzweigten Fettalkoholen handelt es sich um sogenannte Guerbetalkohole, die durch die Guerbet-Synthese zugänglich sind (Ullmann's Enzyclopedia of Industrial Chemistry, 5th Edition, Vol A 10, p. 288).

Von besonderem Interesse sind solche Phosphorsäureester, bei denen der Rest R eine Mischung aus verzweigten und unverzweigten Alkylresten darstellt, beispielsweise eine Mischung aus 0,1 bis 5 % β-verzweigtem Alkyl und 99,9 bis 95 % linearem Alkyl.

Die beschriebenen Phosphorsäureestergemische eigenen sich generell zur Herstellung von tensidhaltigen Formulierungen, worin die Phosphorsäureester das Tensid darstellen. Darüberhinaus eigenen sich diese Phosphorsäureestergemische besonders auch als Emulgator für wasserhaltige Emulsionen vom Öl-in-Wasser oder Wasser-in-Öl-Typ, beispielsweise Emulsionen kosmetischer oder pharmazeutischer Art, bevorzugt auch zur Herstellung alkoholfreier Emulsionen. Diese Emulsionen enthalten die beschriebenen Phosphorsäurealkylester, insbesondere Phosphorsäureestergemische, in Mengen von 0,1 bis 5, vorzugsweise 0,3 bis 3 Gew.-%.

Der Emulsionstyp läßt sich durch Verwendung unterschiedlicher Arten von Alkylaminen bei der Salzbildung beeinflussen. So begünstigen langkettige, lineare primäre Alkylamine die Bildung von Wasser-in-Öl Emulsionen, während kurzkettige verzweigte sek. oder tert. Alkylamine die Bildung von Öl-in-Wasser Emulsionen begünstigen. Die Herstellung solcher wasserhaltiger Emulsionen mit einem Gehalt an Phosphorsäurealkylester erfolgt bevorzugt in der Weise, daß man die Bestandteile der Ölphase zusammen mit dem Gemisch der Phosphorsäurealkylester in Form der freien Säure (d.h. X und Y = H) und der zur Bildung des Salzes äquivalenten Menge eines Alkylamins wie oben angegeben erwärmt und homogen vermischt. Die Bestandteile der Wasserphase werden ebenfalls vermischt und Öl- und Wasserphase werden dann unter intensivem Rühren miteinander gemischt. Anschließend wird der Ansatz kaltgerührt. Anstelle des reinen Alkylaminsalzes kann auch ein Gemisch aus Alkylaminsalz und Alkalisalz vorliegen. Zu diesem Zweck nimmt man eine unterstöchiometrische Menge an Alkylamin - bezogen auf den Phosphorsäurealkylester in der Säureform - und gibt die fehlende Menge an Base in Form eines Alkalihydroxids zu. Auf diese Weise lassen sich bis zu 75 mol%, insbesondere bis zu 25 mol% Alkylamin durch Alkali ersetzen. Anstelle von Alkali kann man auch mit Alkanolamin (z.B. Diethanolamin) oder basischen Aminosäuren (z.B. Arginin, Lysin, Ornithin) arbeiten.

In einer bevorzugten Ausführungsform enthalten die erfindungsgemäßen Emulsionen Phosphorsäurealkylester, in denen X und Y C₁₄-C₁₈-Alkylammonium bedeuten.

In einer weiteren bevorzugten Ausführungsform enthalten die erfindungsgemäßen Emulsionen einen Phosphorsäurealkylester, in dem X und/oder Y ein Gemisch aus C₁₂-C₂₂-Alkylammonium und Alkali-Kation bedeutet.

Die beschriebenen Emulgatoren zeichnen sich durch eine starke Erniedrigung der Grenzflächenspannung auch bei hoher Temperaturbelastung gegenüber polaren und unpolaren Bestandteilen aus. Die zum Teil flüssigen Emulgatoren zeigen eine verbesserte Stabilität gegenüber Elektrolytzusätzen und Säuren und eine hohe Lagerstabilität. Sie liegen im pH-Bereich 5 bis 7 vor und können somit als sehr hautfreundliche Emulgatoren sowohl in Öl-in-Wasser als auch in Wasser-in-Öl-Emulsionen eingesetzt werden, bevorzugt in Hautpflegemitteln.

Der nichtwäßrige Anteil der erfindungsgemäßen Emulsionen, der sich weitgehend aus dem Emulgator- und dem Ölkörper zusammensetzt liegt üblicherweise bei 5 bis 95 % und vorzugsweise 15 bis 75 Gew.-%. Das bedeutet, daß die Emulsionen 5 bis 95 und vorzugsweise 25 bis 85 Gew.-% Wasser enthalten können, abhängig davon, ob Lotionen mit einer vergleichsweise niedrigen oder Cremes und Salben mit hoher Viskosität hergestellt werden sollen.

Als Ölkörper kommen beispielsweise Guerbetalkohole mit 6 bis 18, vorzugsweise 8 bis 10 Kohlenstoffatomen, Ester von linearen C₆-C₁₃-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. aromatische Kohlenwasserstoffe in Betracht.

Die erfindungsgemäßen Emulsionen können als Hautpflegemittel, wie beispielsweise Tagescremes, Nachtcremes, Pflegecremes, Nährcreme, Bodylotions, Salben und dergleichen eingesetzt werden und als weitere Hilfs- und Zusatzstoffe, Co-Emulgatoren, Überfettungsmittel, Fette, Wachse, Stabilisatoren, biogene Wirkstoffe, Glycerin, Konservierungsmittel, Farb- und Duftstoffe enthalten.

Wesentlich für die Erfindung ist, daß die beschriebenen Mischungen der Phosphorsäureester auch ohne Mitverwendung eines nicht-ionischen Tensids als Co-Emulgator eingesetzt werden können. Die Mitverwendung von Co-Emulgatoren ist daher nicht zwingend, aber möglich.

Als nichtionogene O/W-Co-Emulgatoren kommen in Betracht Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; C₁₂-C₁₈-Fettsäuremono- und -diester von Anlagerungsprodukten von 1 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester wie z.B. Polyglycerinpolyricinoleat und Polyglycerinpoly-12-hydroxystearat. Ebenfalls geeignet sind Gemische von Verbindungen aus mehreren dieser Substanzklassen. Die Anlagerungsprodukte von Ethylenoxid und/oder Propylenoxid an Fettalkohole, Fettsäuren, Alkylphenole, Glycerin-mono- und - diester sowie Sorbitanmono- und -diester von Fettsäuren oder an Rizinusöl stellen bekannte, im Handel erhältliche Produkte dar. Es handelt sich dabei um Homologengemische, deren mittlerer Alkoxilierungsgrad dem Verhältnis der Stoffmengen von Ethylenoxid und/oder Propylenoxid und Substrat, mit denen die Anlagerungsreaktion durchgeführt wird, entspricht. C₁₂-C₁₈-Fettsäuremono- und -diester von Anlagerungsprodukten von Ethylenoxid an Glycerin sind aus DE-20 24 051 als Rückfettungsmittel für kosmetische Zubereitungen bekannt.

Als Überfettungsmittel können Substanzen wie beispielsweise polyethoxilierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol in Frage. Als Stabilisatoren können Metallsalze von Fettsäuren, wie z.B. Magnesium-, Aluminium- und/oder Zinkstearat eingesetzt werden. Unter biogenen Wirkstoffen sind beispielsweise Pflanzenextrakte und Vitaminkomplexe zu verstehen. Als Konservierungsmittel eignen sich beispielsweise Phenoxyethanol, Formaldehydlösung, Parabene, Pentandiol oder Sorbinsäure. Als Perlglanzmittel kommen beispielsweise Glycoldistearinsäureester wie Ethylenglycoldistearat, aber auch Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S. 81 - 106, zusammengestellt sind.

Der Gesamtanteil der Hilfs- und Zusatzstoffe kann 1 bis 10, vorzugsweise 2 bis 5 Gew.-%, bezogen auf das Mittel, betragen.
Die Herstellung der Mittel kann in an sich bekannter Weise, d.h. beispielsweise durch Heiß-, Heiß-Heiß/Kalt- bzw. PIT-Emulgierung erfolgen.

### Beispiele

Alle Prozentangaben sind als Gew.-% zu verstehen.

### Beispiel 1: O/W-Creme, pH 5.5

### Zusammensetzung:

| | | |
|---|---|---|
| A | Gemisch aus Mono- und Di-Stearylphosphorsäure | 0.50 % |
| | Paraffinöl Perliquidum | 10.00 % |
| | ®Myritol 318 | 5.00 % |
| | ®Cutina GMS | 3.60 % |
| | ®Cutina FS 45 | 3.60 % |
| | ®Lanette 16 | 0.90 % |
| | Dicyclohexylamin | 0.30 % |
| | NaOH (10 %ig) | 0.50 % |
| B | dem. Wasser | ad 100 % |
| | Glycerin 86 - 88 %ig | 5.00 % |
| | NaOH 10 %ig | q.s. |
| C | ®Euxyl K 400 | 0.15 % |
| | Paraffinöl | 0.30 % |

### Herstellung:

Phase A auf 80 bis 85°C erwärmen
Phase B auf 80 bis 85°C erwärmen
Phase B unter Rühren zu Phase A geben
Entstandene Emulsion kaltrühren
Bei ca. 40°C Phase C zugeben und gut verrühren
Bei ca. 30°C Emulsion homogenisieren

### Beispiel 2: O/W-Creme, pH 5.5

### Zusammensetzung

| | | |
|---|---|---|
| A | Gemisch aus Mono- und Di-Stearylphosphorsäure | 0.50 % |
| | Paraffinöl Perliquidum | 10.00 % |
| | Myritol 318 | 5.90 % |
| | Cutina GMS | 3.60 % |
| | Lanette 16 | 4.50 % |
| | Dicyclohexylamin | 0.30 % |
| | NaOH (10 %ig) | 0.50 % |
| B | dem. Wasser | ad 100 % |
| | Glycerin 86 - 88 %ig | 5.00 % |
| | NaOH 10 %ig | q.s. |
| C | Euxyl K 400 | 0.15 % |
| | Paraffinöl | 0.30 % |

### Herstellung:

Phase A auf 80 bis 85°C erwärmen
Phase B auf 80 bis 85°C erwärmen
Phase B unter Rühren zu Phase A geben
Entstandene Emulsion kaltrühren
Bei ca. 40°C Phase C zugeben und gut verrühren
Bei ca. 30°C Emulsion homogenisieren

### Beispiel 3: Öl-in-Wasser Lotion, pH 5.5

### Zusammensetzung

| | | |
|---|---|---|
| A | Gemisch aus Mono- und Di-Stearylphosphorsäure | 0.50 % |
| | Paraffinöl Perliquidum | 10.00 % |
| | Myritol 318 | 5.80 % |
| | ®Hostacerin DGS | 7.20 % |
| | Di-decylamin | 0.29 % |
| | NaOH (10 %ig) | 0.50 % |
| B | dem. Wasser | ad 100 % |
| | Glycerin 86 - 88 %ig | 5.00 % |
| | NaOH 10 %ig | q.s. |
| C | ®Euxyl K 400 | 0.15 % |
| | Paraffinöl | 0.30 % |

### Herstellung:

Phase A auf 80 bis 85°C erwärmen
Phase B auf 80 bis 85°C erwärmen
Phase B unter Rühren zu Phase A geben
Entstandene Emulsion kaltrühren
Bei ca. 40°C Phase C zugeben und gut verrühren
Bei ca. 30°C Emulsion homogenisieren

### Beispiel 4: Öl-in-Wasser weiche Creme, pH 5.5

### Zusammensetzung

| | | |
|---|---|---|
| A | Gemisch aus Mono- und Di-Stearylphosphorsäure | 0.50 % |
| | Paraffinöl Perliquidum | 10.00 % |
| | Myritol 318 | 5.90 % |
| | Cutina GMS | 3.60 % |
| | ®Prisorine 3515 | 4.50 % |
| | Di-decylamin | 0.29 % |
| | NaOH (10 %ig) | 0.50 % |
| B | dem. Wasser | ad 100 % |
| | Glycerin 86 - 88 %ig | 5.00 % |
| | NaOH 10 %ig | q.s. |
| C | Euxyl K 400 | 0.15 % |
| | Paraffinöl | 0.30 % |

### Herstellung:

Phase A auf 80 bis 85°C erwärmen
Phase B auf 80 bis 85°C erwärmen
Phase B unter Rühren zu Phase A geben
Entstandene Emulsion kaltrühren
Bei ca. 40°C Phase C zugeben und gut verrühren
Bei ca. 30°C Emulsion homogenisieren

### Beispiel 5: Wasser-in-Öl-Rezeptur

### Zusammensetzung

| | | |
|---|---|---|
| A | Gemisch aus Mono- und Di-Stearylphosphorsäure | 1.00 % |
| | Paraffinöl Perliquidum | 12.00 % |
| | Myritol 318 | 10.00 % |
| | ®Forlan L | 2.00 % |
| | ®Lunacera alba | 5.00 % |
| | ®Armeen HTD | 0.90 % |
| B | dem. Wasser | 65.65 % |
| | Glycerin 86 - 88 %ig | 3.00 % |
| C | Euxyl K 400 | 0.15 % |
| | Paraffinöl | 0.30 % |

### Herstellung:

Phase A auf 80 bis 85°C erwärmen
Phase B auf 80 bis 85°C erwärmen
Phase B unter Rühren zu Phase A geben
Entstandene Emulsion kaltrühren
Bei ca. 40°C Phase C zugeben und gut verrühren
Bei ca. 30°C Emulsion homogenisieren

### Beispiel 6: Wasser-in-Öl-Rezeptur

### Zusammensetzung

| | | |
|---|---|---|
| A | Gemisch aus Mono- und Di-Stearylphosphorsäure | 1.00 % |
| | Paraffinöl Perliquidum | 12.00 % |
| | Myritol 318 | 12.00 % |
| | Forlan L | 2.00 % |
| | Lunacera alba | 1.00 % |
| | ®Elfacos ST 9 | 2.00 % |
| | ®Genamin 18 R 100 D | 0.70 % |
| B | dem. Wasser | 65.85 % |
| | Glycerin 86 - 88 %ig | 3.00 % |
| C | Euxyl K 400 | 0.15 % |
| | Paraffinöl | 0.30 % |

### Herstellung:

Phase A auf 80 bis 85°C erwärmen
Phase B auf 80 bis 85°C erwärmen
Phase B unter Rühren zu Phase A geben
Entstandene Emulsion kaltrühren
Bei ca. 40°C Phase C zugeben und gut verrühren
Bei ca. 30°C Emulsion homogenisieren

### Verzeichnis der eingesetzten Handelsprodukte:

| Paraffin perliquidium (Wagner, Bremen) Mineralöl | |
|---|---|
| Myritol 318 (Henkel, Düsseldorf) | Caprin/Capryltriglycerid |
| Cutina GMS (Henkel) | Glycerinstearat |
| Cutina FS (Henkel) | Palmitinsäure, Stearinsäure |
| Lanette 16 (Henkel) | Cetylalkohol |
| Lanette O (Henkel) | Cetearylalcohol |
| Hostacerin DGS (Clariant) | Polyglycerin-2-PEG-4-Stearat |
| Prisorine 3515 (Unichema) | Isostearylalkohol |
| Glycerin 86 bis 88 % (Henkel) | Glycerin |
| Euxyl K 400 (Schülke + Mayr) | Methyldibromglutaronitril |
| Forlan L (Erbslöh, Krefeld) | Lanolin, hydriertes Cocosöl, Sorbitan Sesquioleat, Stearylalcohol, Cetylalkohol |
| Lunacera alba (Fuller, Lüneburg) | Cera Alba |
| Armeen HTD (Akzo, Düren) | hydriertes Talgfettamin |
| Elfacos ST 9 (Akzo, Düren) | PEG-45 / Dodecyl Glycol Copolymer |
| Genamin 18R 100D (Clariant) | Octadecylamin |

## Patentansprüche

1. Kosmetische und pharmazeutische Emulsionen, enthaltend als Emulgator Phosphorsäurealkylester der Formel 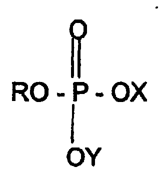 worin R C₁₂-C₂₂-, vorzugsweise C₁₄-C₁₈-Alkyl bedeutet, X eine Gruppe der Formeln NH₃R¹, NH₂R²R³, NHR⁴R⁵R⁶ oder ein Gemisch aus bis zu 75 Mol-% dieser Alkylamine und einem Alkali-Kation bedeutet, Y die gleiche Bedeutung wie R oder die gleiche Bedeutung wie X hat und R¹ C₈-C₂₂-Alkyl, R² und R³ C₁-C₂₂-Alkyl oder Cyclohexyl, R⁴, R⁵ und R⁶ C₁-C₄-Alkyl bedeuten.

2. Kosmetische und pharmazeutische Emulsionen nach Anspruch 1, **dadurch gekennzeichnet, dass** der Phosphorsäuralkylester 0 bis 10 Gew.-% des korrespondierenden Tri-esters enthält.

3. Kosmetische und pharmazeutische Emulsionen nach Anspruch 1, **dadurch gekennzeichnet, dass** R in dem Phosphorsäurealkylester C₁₄-C₁₈-Alkyl ist.

4. Kosmetische und pharmazeutische Emulsionen nach Anspruch 1, **dadurch gekennzeichnet, dass** X und Y in dem Phosphorsäurealkylester C₁₄-C₁₈-Alkylammonium bedeuten.

5. Kosmetische und pharmazeutische Emulsionen nach Anspruch 1, **dadurch gekennzeichnet, dass** R in dem Phosphorsäurealkylester eine Mischung aus geradkettigen und β-verzweigten Alkylresten ist.

6. Kosmetische und pharmazeutische Emulsionen nach Anspruch 1, **dadurch gekennzeichnet, dass** sie 0,1 bis 5 Gew.-% des Phosphorsäurealkylesters enthalten.

7. Kosmetische und pharmazeutische Emulsionen nach Anspruch 1, **dadurch gekennzeichnet, dass** X und/oder Y in dem Phosphorsäurealkylester ein Gemisch aus C₁₂-C₂₂-Alkylammonium und Alkali-Kation bedeutet.

## Claims

1. A cosmetic or pharmaceutical emulsion comprising, as emulsifier, alkyl phosphate of the formula 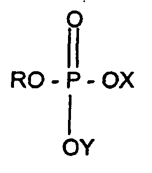 in which R is C₁₂-C₂₂-alkyl, preferably C₁₄-C₁₈-alkyl, X is a group of the formulae NH₃R¹, NH₂R²R³, NHR⁴R⁵R⁶ or is a mixture of up to 75 mol% of these alkylamines and an alkali metal cation, Y is as defined for R or is as defined for X, and R¹ is C₈-C₂₂-alkyl, R² and R³ are C₁-C₂₂-alkyl or cyclohexyl, and R⁴, R⁵ and R⁶ are C₁-C₄-alkyl.

2. A cosmetic or pharmaceutical emulsion as claimed in claim 1, wherein the alkyl phosphate comprises from 0 to 10% by weight of the corresponding triester.

3. A cosmetic or pharmaceutical emulsion as claimed in claim 1, wherein R in the alkyl phosphate is C₁₄-C₁₈-alkyl.

4. A cosmetic or pharmaceutical emulsion as claimed in claim 1, wherein X and Y in the alkyl phosphate are C₁₄-C₁₈-alkylammonium.

5. A cosmetic or pharmaceutical emulsion as claimed in claim 1, wherein R in the alkyl phosphate is a mixture of straight-chain and β-branched alkyl radicals.

6. An cosmetic or pharmaceutical emulsion as claimed in claim 1 the comprising from 0.1 to 5% by weight of the alkyl phosphate.

7. A cosmetic or pharmaceutical emulsion as claimed in claim 1, wherein X and/or Y in the alkyl phosphate is a mixture of C₁₂-C₂₂-alkylammonium and alkali metal cation.

## Revendications

1. Emulsions cosmétiques et pharmaceutiques, contenant comme agent émulsionnant des esters alkyliques d'acide phosphorique de formule 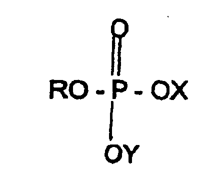 dans laquelle R représente un groupe alkyle en C₁₂ à C₂₂, de préférence en C₁₄ à C₁₈, X représente un groupe des formules NH₃R¹, NH₂R²R³, NHR⁴R⁵R⁶ ou un mélange de jusqu'à 75 % en mole de cette alkylamine et d'un cation alcalin, Y a une signification identique à R ou une signification identique à X et R¹ représente un groupe alkyle en C₈ à C₂₂, R² et R³ un groupe alkyle en C₁ à C₂₂ ou cyclohexyle, R⁴, R⁵ et R⁶ un groupe alkyle en C₁ à C₄.

2. Emulsions cosmétiques et pharmaceutiques selon la revendication 1, **caractérisées en ce que** l'ester alkylique d'acide phosphorique contient de 0 à 10 % en poids du tri-ester correspondant.

3. Emulsions cosmétiques et pharmaceutiques selon la revendication 1, **caractérisées en ce que** R dans l'ester alkylique d'acide phosphorique est un groupe alkyle en C₁₄ à C₁₈.

4. Emulsions cosmétiques et pharmaceutiques selon la revendication 1, **caractérisées en ce que** X et Y dans l'ester alkylique d'acide phosphorique représentent un groupe alkylammonium en C₁₄ à C₁₈.

5. Emulsions cosmétiques et pharmaceutiques selon la revendication 1, **caractérisées en ce que** R dans l'ester alkylique d'acide phosphorique est un mélange de résidus alkyle à chaîne linéaire et β-ramifiée.

6. Emulsions cosmétiques et pharmaceutiques selon la revendication 1, **caractérisées en ce qu'**elles contiennent de 0,1 à 5 % en poids de l'ester alkylique d'acide phosphorique.

7. Emulsions cosmétiques et pharmaceutiques selon la revendication 1, **caractérisées en ce que** X et/ou Y dans l'ester alkylique d'acide phosphorique représente un mélange d'alkylammonium en C₁₂ à C₂₂ et d'un cation alcalin.
